# Europäisches Patentamt
# European Patent Office
# Office européen des brevets

(11) Veröffentlichungsnummer: **0 217 226 B1**

(12) **EUROPÄISCHE PATENTSCHRIFT**

(45) Veröffentlichungstag der Patentschrift:
12.04.89

(21) Anmeldenummer: **86112807.2**

(22) Anmeldetag: **16.09.86**

(51) Int. Cl.⁴: **A61B 6/06, G21K 1/02**

(54) Kollimator für Strahlendiagnostikgeräte.

(30) Priorität: **30.09.85 DE 3534893**

(43) Veröffentlichungstag der Anmeldung:
**08.04.87 Patentblatt 87/15**

(45) Bekanntmachung des Hinweises auf die Patenterteilung:
**12.04.89 Patentblatt 89/15**

(84) Benannte Vertragsstaaten:
**DE FR**

(56) Entgegenhaltungen:
**FR-A- 2 260 014**
**US-A- 3 566 175**

(73) Patentinhaber: **Siemens Aktiengesellschaft Berlin und München, Wittelsbacherplatz 2, D-8000 München 2(DE)**

(72) Erfinder: **Klein, Sigismund, Hassfurter Strasse 8, D-8500 Nürnberg(DE)**

ACTORUM AG

## Beschreibung

Die Erfindung betrifft einen Kollimator für ein Strahlendiagnostikgerät mit zwei parallelen, im Abstand voneinander angeordneten Trägerteilen, die eine Reihe von auf den Strahlenfokus gerichteten Schlitzen aufweisen, in denen Kollimatorbleche aus strahlungsabsorbierendem Material eingesetzt sind.

In der DE-PS 2 840 965 ist ein Strahlendiagnostikgerät beschrieben, das mit Hilfe der Strahlenabsorption von Röntgenstrahlung die Erzeugung von Schichtbildern eines Aufnahmeobjektes erlaubt. Den quantitativen Nachweis der das Aufnahmeobjekt durchstrahlten Röntgenstrahlung erbringen viele hundert Einzeldetektorelemente, die auf Kollimatorblechen befestigt und durch Schlitze in zwei parallel auf Abstand angeordneten Trägerteilen gehalten werden. Die Schlitze sind dabei auf den Strahlenfokus ausgerichtet. In der Abbildung 5 der DE-PS 2 840 965 ist ein Kollimatorblech 3a mit dem auf ihm befestigten Einzeldetektorelement 2a gezeigt.

Bei dieser Ausführungsform ergibt sich die Möglichkeit, daß die Kollimatorbleche nicht genügend in ihrer gewünschten Position fixiert sind, falls die Schlitze aufgrund fertigungstechnischer Unzulänglichkeiten eine grössere Breite besitzen als es der Dicke eines Kollimatorbleches entspricht. Dies hat zur Folge, daß die Kollimatorbleche innerhalb der Schlitze so viel Spiel haben, daß ihre Ausrichtung auf den Strahlenfokus nicht mehr besteht. Abschattung und eine verminderte Nutzung der wirksamen Detektorfläche sind die Folge.

Der Erfindung liegt die Aufgabe zugrunde, einen Kollimator für ein Strahlendiagnostikgerät der eingangs genannten Art so auszubilden, daß die Kollimatorbleche auch bei größeren Schlitzweiten spielfrei in den Schlitzen und stets auf den Strahlenfokus gerichtet bleiben.

Die Aufgabe ist erfindungsgemäß dadurch gelöst, daß die Kollimatorbleche an ihren in die Schlitze eingesetzten Kanten über die Dicke des Bleches hinausragende, federnde Blechzungen aufweisen.

Diese federnden Zungen stützen sich selbst an jeweils einer Innenwandung der Schlitze ab und drücken die Bleche an die jeweils gegenüberliegende Innenwandung, so daß auf diese Weise die Bleche spielfrei in den betreffenden Schlitzen gehalten werden. Die Schlitze können mit größerer Toleranz breiter gesägt oder gefräst werden.

Die Erfindung ist nachfolgend anhand eines Ausführungsbeispieles und der Zeichnung näher erläutert. Es zeigen:

Fig. 1 die Vorderansicht eines Kollimatorbleches mit federnden Zungen nach der Erfindung,
Fig. 2 die Seitenansicht eines Kollimatorbleches mit federnden Zungen nach der Erfindung, und
Fig. 3 ein in ein Schlitzpaar eingesetztes Kollimatorblech nach der Erfindung.

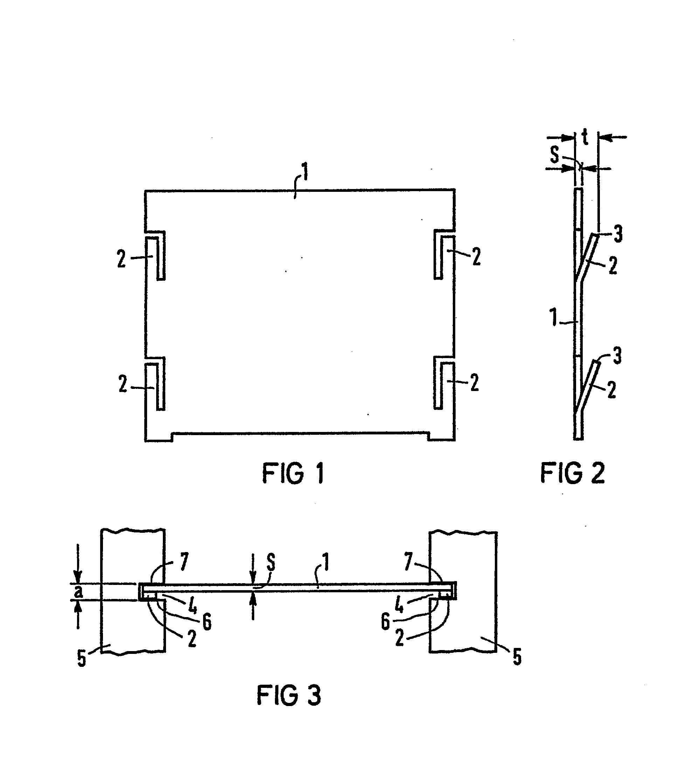

Die Figur 1 zeigt eine mögliche Ausführungsform des erfindungsgemäßen Kollimatorbleches in der Vorderansicht. An den seitlichen Kanten eines Kollimatorbleches 1, die bei seinem Einsetzen in die Trägerteile durch die Schlitzinnenwandungen eingeschlossen werden, sind durch Sägen oder Fräsen rechteckige Metallzungen 2 herausgearbeitet. Diese Metallzungen 2 sind dann, wie in der Seitenansicht (Fig. 2) ersichtlich, aus der Kollimatorblechebene herausgebogen. Während das Kollimatorblech lediglich die Dicke S aufweist, wird aufgrund der herausstehenden Enden 3 der federnden Metallzungen 2 eine größere Breite t in Anspruch genommen.

Ein in zwei fokussiert gesägte Schlitze 4 zweier gegenüberliegender Trägerteile 5 eingesetztes Kollimatorblech 1 zeigt die Figur 3. Man wählt dabei die Schlitzbreite a maximal so groß, daß sie die beanspruchte Breite t des Kollimatorbleches 1 nicht überschreitet. Dadurch sind bei eingesetztem Kollimatorblech 1 die Metallzungen 2 gespannt. Sie liegen jeweils an einer Innenwandung 6 des Schlitzes 4 und drücken das Kollimatorblech 1 gegen die jeweils gegenüberliegende Innenwandung 7 an. Auf diese Weise wird das Kollimatorblech 1 stets spielfrei in den Schlitzen 4 gehalten.

Ein zusätzlicher Vorteil dieser Ausführungsform ergibt sich durch eine leichte Auswechselbarkeit der Bleche. Würden die Bleche in die Schlitze eingeklebt, so wäre die Genauigkeit der Fokussierung durch die Blech- und Schlitztoleranz begrenzt. Bei Verwendung der erfindungsgemäßen Kollimatorbleche können Schlitze mit größerer Breite a und größerer Toleranz in Kauf genommen werden.

## Patentansprüche

1. Kollimator für ein Strahlendiagnostikgerät mit zwei parallelen, im Abstand voneinander angeordneten Trägerteilen (5), die eine Reihe von auf den Strahlenfokus gerichteten Schlitzen (4) aufweisen, in denen Kollimatorbleche (1) aus strahlungsabsorbierendem Material eingesetzt sind, dadurch gekennzeichnet, daß die Kollimatorbleche (1) an ihren in die Schlitze (4) eingesetzten Kanten über die Dicke des Bleches (1) hinausragende, federnde Blechzungen (2) aufweisen, die an jeweils einer Innenwandung (6) der Schlitze (4) anliegen und die Kollimatorbleche (1) gegen die jeweils gegenüberliegende Innenwandung (7) drücken.

2. Kollimator nach Anspruch 1, dadurch gekennzeichnet, daß die aufgrund der federnden Blechzungen (2) beanspruchte Breite der Kollimatorbleche (1) bei nicht-gespannten Blechzungen (2) die Breite der Schlitze (4) übertrifft.

## Claims

1. A collimator for a diagnostic irradiation apparatus having two supporting parts (5) arranged parallel to and spaced from one another which have a row of slits (4) directed towards the focus of the radiation, in which collimator sheets (1) of radiation absorbing material are inserted, characterised in that the collimator sheets (1) have at their edges inserted in the slits (4) resilient sheet tongues (2) projecting beyond the thickness of the sheet (1) which each bear against an inner wall (6) of the slit (4) and press the collimator sheets (1) aginst the respective opposed inner wall (7).

2. A collimator according to claim 1, characterised in that when the sheet tongues (2) are not stressed the width of the collimator sheets (1) loaded owing to the resilient sheet tongues (2) exceeds the width of the slits (4).

**Revendications**

1. Collimateur pour un appareil de radiodiagnostic comportant deux éléments de support parallèles (5), situés à distance l'un de l'autre et possédant une série de fentes (4), qui sont orientées sur le foyer du rayonnement et dans lesquelles sont insérées des plaques collimatrices (1) réalisées en un matériau absorbant le rayonnement, caractérisé par le fait que les plaques collimatrices (5) possèdent, sur leurs bords insérés dans les fentes (4), des languettes élastiques (2), qui font saillie au delà de l'épaisseur des plaques (1), s'appliquent contre les parois intérieures respectives (6) des fentes (4) et repoussent les plaques collimatrices (1) contre la paroi intérieure (7) située respectivement en vis-à-vis.

2. Collimateur suivant la revendication 1, caractérisé par le fait que la largeur des plaques collimatrices (1), qui est nécessaire en raison de la présence des languettes élastiques (2), ne dépasse pas la largeur des fentes (4) lorsque les languettes (2) ne sont pas placées sous contrainte.

FIG 1

FIG 2

FIG 3